# EUROPÄISCHE PATENTANMELDUNG

(11) **EP 1 637 513 A2**
(43) Veröffentlichungstag der Anmeldung: **22.03.2006**
(21) Anmeldenummer: 05014742.0
(22) Anmeldetag: 07.07.2005
(51) Int. Cl.: C07C 45/77, C07C 49/92

(54) **Herstellung von Ytterbium(III)-Beta-diketonaten**

(30) Priorität: 20.07.2004 DE 102004035129
(71) Anmelder: LANXESS Deutschland GmbH, 51369 Leverkusen (DE)
(72) Erfinder: Gerlach, Arne, Dr., 65843 Sulzbach (DE); Groth, Torsten, Dr., 51519 Odenthal (DE); Hofacker, Steffen, Dr., 51519 Odenthal (DE)

(57) **Zusammenfassung**

Die Erfindung betrifft ein Verfahren zur Herstellung von Ytterbium(III)-β-diketonaten, insbesondere Ytterbium(III)-acetylacetonat, die nach diesem Verfahren hergestellten Ytterbium(III)-β-diketonate sowie deren Verwendung als Katalysatoren.

## Beschreibung

Die Erfindung betrifft ein Verfahren zur Herstellung von Ytterbium(III)-β-diketonaten, insbesondere Ytterbium(III)-acetylacetonat, die nach diesem Verfahren hergestellten Ytterbium(III)-β-diketonate sowie deren Verwendung als Katalysatoren.

Oligocarbonatpolyole sind wichtige Vorprodukte, beispielsweise bei der Herstellung von Kunststoffen, Lacken und Klebstoffen. Dazu werden sie beispielsweise mit Isocyanaten, Epoxiden, (cyclischen) Estern, Säuren oder Säureanhydriden umgesetzt. Oligocarbonatpolyole können prinzipiell aus aliphatischen Polyolen durch Umsetzung mit Phosgen, bis-Chlorkohlensäureestern, Diarylcarbonaten, cyclischen Carbonaten oder Dialkylcarbonaten hergestellt werden.

Bei Umsetzung mit Alkylcarbonaten, wie z.B. Dimethylcarbonat, werden häufig Umesterungskatalysatoren eingesetzt, z.B. Alkali- oder Erdalkali-Metalle sowie deren Oxide, Alkoxide, Carbonate, Borate oder Salze organischer Säuren (z.B. WO 2003/2630).

Darüber hinaus werden bevorzugt Zinn oder Zinn-organische Verbindungen wie Bis(tri-butylzinn)oxid, Dibutylzinnlaurat oder aber auch Dibutylzinnoxid (DE-A 2 523 352) sowie Verbindungen des Titans wie Titantetrabutylat, Titantetraisopropylat oder Titandioxid als Umesterungskatalysatoren verwendet (z.B. EP-B 0 343 572, WO 2003/2630).

Die aus dem Stand der Technik bekannten Umesterungskatalysatoren zur Herstellung von aliphatischen Oligocarbonatpolyolen durch Umsetzung von Alkylcarbonaten mit aliphatischen Polyolen weisen aber einige Nachteile auf wie z.B. das Verbleiben Zinn-organischer Verbindungen als potentieller Cancerogene in Folgeprodukten der Oligocarbonatpolyole, zusätzlich erforderliche Neutralisationsschritte bei Verwendung von starken Basen wie Alkali- oder ErdalkaliMetallen oder deren Alkoxiden als Katalysatoren oder unerwünschte Verfärbungen (Gelbfärbung) bei der Lagerung bei Einsatz von Ti-Verbindungen als Katalysatoren. Titan-haltige Katalysatoren besitzen weiterhin den Nachteil einer hohen Aktivität gegenüber Isocyanatgruppen-haltigen Verbindungen bei der weiteren Umsetzung der hydoxylterminierten Oligocarbonate als Polyurethanrohstoff auf, so dass zur Vermeidung dieses Nachteils ein weiterer Inaktivierungsschritt für den im Produkt verbleibenden Umesterungskatalysator erforderlich ist. Bei dieser Inaktivierung beispielsweise durch Zusatz von Phosphorsäure (EP-B 1 091 993) oder Hydrolyse der Titanverbindungen (US-A 4 891 421) wird dem Produkt eine entsprechende Menge Wasser zugesetzt, die nach erfolgter Desaktivierung destillativ wieder aus dem Produkt entfernt werden muss. Weiterhin ist es mit den bisher verwendeten Katalysatoren nicht möglich gewesen, die Reaktionstemperatur, die üblicherweise zwischen 150°C und 230°C liegt, zu senken, um die Bildung von Nebenprodukten wie Ethern oder Vinylgruppen, die bei erhöhter Temperatur entstehen können, weitgehend zu vermeiden. Diese unerwünschten Endgruppen führen als Kettenabbrecher für nachfolgende Polymerisationsreaktionen, z.B. im Falle der Polyurethanreaktion mit mehrfachfunktionellen (Poly)Isocyanaten, zu einer Erniedrigung der Netzwerkdichte und damit zu schlechteren Produkteigenschaften (z.B. Lösemittel- oder Säurebeständigkeit). Darüber hinaus weisen Oligocarbonatpolyole, die mit Hilfe der aus dem Stand der Technik bekannten Katalysatoren hergestellt wurden, hohe Gehalte an Ethergruppen (z.B. Methyl-, Hexylether etc.) auf, die in den Oligocarbonatpolyolen z.B. zu unzureichender Heißluftbeständigkeit von Gießelastomeren führen, da Etherbindungen im Material unter diesen Bedingungen gespalten werden und so zu einem Versagen des Materials führen.

DE-A 101 56 896 lehrt, dass die vorangehend aufgeführten Nachteile durch die Verwendung von Selten-Erd-Metallverbindungen beseitigt werden können. Mögliche Selten-Erd-Metallverbindung stellen Ytterbium(III)-β-diketonate, insbesondere Ytterbium(III)acetylacetonat dar.

In der Literatur sind mehrere Verfahren zur Herstellung von Lanthanid(III)acetylacetonaten beschrieben. So beschreiben z.B. J. G. Stites et al. (*J. Am. Chem. Soc.,.***1948***, 70,* 3142-3143) die Herstellung von Lanthanid(III)acetylacetonaten aus Lanthanid(III)chloriden mit Acetylaceton und Ammoniak in Wasser. Ein wasserfreies Verfahren zur Herstellung von Ytterbium(III)acetylacetonat unter Verwendung von gasförmigem Ammoniak und organischen Lösungsmitteln ist in DT-A 25 55 556 A1 beschrieben.

Setzt man jedoch nach einem dieser Verfahren hergestelltes Ytterbium(III)acetylacetonat ein, so zeigt sich, dass bei der Verwendung dieses Materials z.B. als Umesterungskatalysator für die Reaktion von organischen Carbonatestern mit aliphatischen Polyolen zwar eine katalytische Aktivität nachgewiesen werden kann, diese aber hinsichtlich der Raum-Zeit-Ausbeute für eine kommerzielle Herstellung von aliphatischen Oligocarbonaten nicht zufriedenstellend ist.

Aufgabe der vorliegenden Erfindung war daher, Selten-Erd-Metallverbindungen, wie z.B. Ytterbium(III)-β-diketonate, bereitzustellen, die z.B. als Umesterungskatalysatoren geeignet sind und eine gegenüber dem Stand der Technik erhöhte katalytische Wirksamkeit aufweisen. Eine weitere Aufgabe der vorliegenden Erfindung lag darin, insbesondere Ytterbium(III)acetylacetonat bereitzustellen, welches eine gegenüber dem Stand der Technik erhöhte katalytische Wirksamkeit aufweist.

Überraschend wurde nun gefunden, dass Ytterbium(III)-β-diketonate, die aus einem Ytterbium(III)-Salz, einem 1,3-Diketon und einer Base in wässriger Lösung hergestellt wurden, diese Anforderung erfüllen, wenn weniger als 3 Äquivalente 1,3-Diketon bezogen auf 1 Äquivalent Ytterbium(III)-Salz bei der Umsetzung eingesetzt werden und das erhaltene Ytterbium(III)-β-diketonat bei wenigstens einer Temperatur von 45°C bis 150°C länger als 5 Stunden getrocknet wird.

Gegenstand der vorliegenden Erfindung ist daher ein Verfahren zur Herstellung eines Ytterbium(III)-β-diketonats durch Umsetzung wenigstens eines Ytterbium(III)-Salzes, wenigstens eines 1,3-Diketons und einer Base in wässriger Lösung, dadurch gekennzeichnet, dass weniger als 3 Äquivalente 1,3-Diketon bezogen auf 1 Äquivalent Ytterbium(III)-Salz eingesetzt werden und das erhaltene Ytterbium(III)-β-diketonat bei wenigstens einer Temperatur von >50°C bis 150°C länger als 5 Stunden getrocknet wird.

Unter einer Base ist im Rahmen der Erfindung bevorzugt eine Brönsted_Base zu verstehen, wobei der pK_{A}-Wert des Säure-Base-Paares in wässriger Lösung bei 25°C bevorzugt kleiner als 12 ist zu verstehen. Bevorzugt werden als Basen Ammoniak, Na₂CO₃, K₂CO₃ oder Natriumacetat verwendet, besonders bevorzugt wird Ammoniak, ganz besonders bevorzugt in Form einer wässrigen Lösung verwendet.

Bevorzugt werden als Ytterbium(III)-Salze Ytterbium(III)halogenide oder Ytterbium(III)nitrat, besonders bevorzugt Ytterbium(III)halogenide eingesetzt, ganz besonders bevorzugt wird Ytterbium(III)chlorid eingesetzt. Die Ytterbium(III)-Salze können in wasserfreier oder hydratisierter Form eingesetzt werden.

Bevorzugt werden als 1,3-Diketon Acetylaceton, Dibenzoylmethan oder Dipivaloylmethan eingesetzt, besonders bevorzugt wird Acetylaceton eingesetzt.

Das erfindungsgemäße Verfahren wird bevorzugt so durchgeführt, dass wenigstens ein Ytterbium(III)-Salz, bevorzugt ein Ytterbium(III)-Salz und wenigstens ein 1,3-Diketon, bevorzugt ein 1,3-Diketon in wässriger Lösung vorgelegt werden und innerhalb einer geeigneten Zeit eine wässrige Ammoniaklösung zugegeben wird. Dabei werden Ytterbium(III)-Salz und 1,3-Diketon bevorzugt bei einer Temperatur von 15°C bis 60°C, bevorzugt jedoch Umgebungstemperatur vorgelegt. Die Zugabe der wässrigen Ammoniaklösung erfolgt vorzugsweise innerhalb eines Zeitraumes von 15 Minuten bis 10 Stunden, besonders bevorzugt 30 Minuten bis 5 Stunden. Die Temperatur steigt bei der Zugabe bevorzugt auf Temperaturen von 15°C bis 60°C. Nach beendeter Zugabe wird die Reaktionsmischung gegebenenfalls noch über einen Zeitraum von 15 min bis 10 Stunden, bevorzugt 30 Minuten bis 5 Stunden nachgerührt und anschließend auf eine Temperatur von 1°C bis < 15°C, bevorzugt 2°C bis 10°C, besonders bevorzugt 3°C bis 5°C abgekühlt. Das Ytterbium(III)-β-diketonat wird - beispielsweise durch Filtration - von der wässrigen Mutterlauge abgetrennt, gegebenenfalls einmal oder mehrfach mit Wasser nachgewaschen und anschließend getrocknet.

Bevorzugt werden 2,5 bis 2,99, besonders bevorzugt 2,6 bis 2,9 Äquivalente 1,3-Diketon bezogen auf 1 Äquivalent Ytterbium(III)-Salz eingesetzt.

Weiterhin bevorzugt wird die Umsetzung des Ytterbium(III)-Salzes mit dem 1,3-Diketon und Ammoniak in der Weise durchgeführt, dass nach der Zugabe der Ammoniaklösung die Reaktionsmischung einen pH-Wert von 5 bis 10, bevorzugt von 6,5 bis 9,5, besonders bevorzugt von 8 bis 9 aufweist. Die pH-Wert-Angaben beziehen sich dabei auf die Werte, die bei der entsprechenden Temperatur der Reaktionsmischung nach Zugabe der Ammoniaklösung gemessen werden. Die Messung erfolgt in der Regel mit einer kommerziellen pH-Elektrode (Glaselektrode).

Die Trocknung erfolgt bei wenigstens einer Temperatur von >50°C bis 150°C, bevorzugt von 80 bis 130°C, besonders bevorzugt von 100°C bis 120°C für mehr als 5 Stunden, bevorzugt für 7 bis 60 Stunden, besonders bevorzugt für 10 bis 48 Stunden.

In einer bevorzugten Ausführungsform erfolgt die Trocknung bei unterschiedlichen Temperaturen in der Weise, dass zunächst bei einer niedrigen Temperatur, die auch ≤50°C sein kann, und anschließend bei einer höheren Temperatur von >50°C bis 150°C, bevorzugt von 80 bis 130°C, besonders bevorzugt von 100°C bis 120°C getrocknet wird. Dabei wird bei der niedrigen Temperatur bevorzugt für 1 bis 10 Stunden, besonders bevorzugt für 3 bis 7 Stunden, bei der höheren Temperatur für mehr als 5 Stunden, bevorzugt für 7 bis 60 Stunden, besonders bevorzugt für 10 bis 48 Stunden getrocknet.

Die Trocknung wird weiterhin bevorzugt bei wenigstens einem Druck ≤1 bar, bevorzugt ≤ 500 mbar, besonders bevorzugt ≤ 100 mbar durchgeführt.

Als wässrige Ammoniaklösungen werden vorzugsweise solche in kommerziell erhältlichen Konzentrationen eingesetzt. Es können aber auch beliebig konzentrierte oder verdünnte wässrige Ammoniaklösungen eingesetzt werden.

Unter wässriger Lösung ist im Rahmen der Erfindung bevorzugt eine Lösung zu verstehen, die als Lösungsmittel Wasser enthält. Eine solche wässrige Lösung kann im Rahmen der Erfindung auch Zusätze von organischen Lösungsmitteln enthalten. Besonders bevorzugt enthält eine wässrige Lösung im Rahmen der Erfindung jedoch als Lösungsmittel im Wesentlichen Wasser.

Die nach dem erfindungsgemäßen Verfahren hergestellten Ytterbium(III)-β-diketonate eignen sich hervorragend als Umesterungskatalysatoren. Beispielsweise weisen sie gegenüber nach bekannten Verfahren hergestelltem Ytterbium(III)acetylacetonat bei der Umesterung von organischen Carbonaten mit aliphatischen Polyolen zur Herstellung aliphatischer Oligocarbonatpolyole eine deutlich erhöhte katalytische Wirksamkeit auf.

Weiterhin Gegenstand der Erfindung sind daher Ytterbium(III)-β-diketonate hergestellt nach dem erfindungsgemäßen Verfahren.

Die erfindungsgemäß hergestellten Ytterbium(III)-β-diketonate eignen sich aufgrund ihrer gegenüber nach bekannten Verfahren hergestellten Ytterbium(III)-β-diketonaten erhöhten katalytischen Aktivität hervorragend als Umesterungskatalysatoren.

Weiterhin Gegenstand der Erfindung ist daher die Verwendung der erfindungsgemäß hergestellten Ytterbium(III)-β-diketonate als Umesterungskatalysatoren.

Die folgenden Beispiele dienen der beispielhaften Erläuterung und Veranschaulichung der Erfindung, stellen jedoch keine Beschränkung dar.

### Beispiele

### Beispiel 1:

Herstellung von Ytterbium(III)acetylacetonat mit einem Acetylaceton/YbCl₃-Verhältnis von 2,8/1,0:
a) In einem 2000 ml Doppelwand-Glasreaktor mit Thermometer und pH-Elektrode wurden 688 g Wasser, 200 g (0.516 mol, 1.0 Äquivalente) Ytterbium(III)chlorid-hexahydrat und 148 g (1.445 mol, 2.8 Äquivalente) Acetylaceton bei Raumtemperatur (20°C) vorgelegt. Innerhalb einer Stunde wurden 105 g (1.600 mol, 3.1 Äquivalente) einer 26 %igen wässrigen Ammoniaklösung zugetropft. Während der Zugabe stieg die Temperatur auf 35°C und der pH-Wert auf 8.09. Es wurde dann noch eine Stunde nachgerührt bevor auf 5°C abgekühlt wurde. Filtration und Waschen mit zweimal 250 ml Wasser lieferte 218 g Rohmaterial. Dieses Rohmaterial wurde anschließend bei 20 mbar 5 h bei 50°C und 36 h bei 100°C getrocknet und die Ausbeute bestimmt (vgl. Tab.1).
b) Es wurde Ytterbium(III)acetylacetonat wie unter a) beschrieben hergestellt mit der Änderung, dass das Rohmaterial bei 20 mbar für 5 h bei 50°C und für 60 h bei 100°C getrocknet wurde.

### Vergleichsbeispiel 1:

Zum Vergleich wurde Ytterbium(III)acetylacetonat wie unter Beispiel 1 a) beschrieben hergestellt mit der Änderung, dass das Rohmaterial bei 20 mbar für 5 h bei 50°C getrocknet wurde.

Nach erfolgter Trocknung wurde jeweils die katalytische Wirksamkeit (Aktivität) des erhaltenen Ytterbium(III)acetylacetonats bestimmt. Die Ergebnisse sind in Tab. 1 aufgeführt.

### Bestimmung der katalytischen Wirksamkeit (Aktivität):

Die Bestimmung der Katalysatoraktivität erfolgte durch Herstellung eines OligocarbonatdiolsHFC 1947A. Als Maß für die Aktivität des Ytterbium(III)acetylacetonats wurde hierzu die Hydroxylzahl (OHZ) nach Herstellung des Oligocarbonatdiols gemäß DIN 53240-2 bestimmt. Im Einzelnen wurde folgender Versuch zur jeweiligen Aktivitätsbestimmung des Ytterbium(III)acetylacetonats aus Beispiel 1 a), b) und Vergleichsbeispiel 1 durchgeführt:
332,1 g 1,6-Hexandiol wurden unter Stickstoffatmosphäre bei 120°C und 20 mbar 2 h entwässert. Danach wurden bei 80°C 352,7 g Dimethylcarbonat und 0,08 g Ytterbium(III)acetylacetonat zugegeben und 24 Stunden unter Rückfluss und Inertgasatmosphäre zur Reaktion gebracht.

Anschließend wurde die Temperatur auf 150°C erhöht und für 4 Stunden Methanol und Dimethylcarbonat aus dem Reaktionsgemisch unter Normaldruck destillativ entfernt. Danach wurde die Temperatur auf 180°C gesteigert und für weiter 4 Stunden Nebenprodukte entfernt. Schließlich wurde die Temperatur auf 130 °C zurückgenommen und der Druck auf < 30 mbar reduziert. Bei gleichzeitigem Durchleiten eines Stickstoffstroms (2 l/h) durch das Reaktionsgemisch wurden weitere Nebenprodukte durch kontinuierliche Erhöhung der Temperatur auf 180°C destillativ entfernt. Die Erhöhung der Temperatur von 130 auf 180°C erfolgte unter der zeitlichen Maßgabe, dass die Kopftemperatur 60°C nicht überstieg. Nach Erreichen von 180°C wurde diese Temperatur bei einem Druck von < 30 mbar für 4 Stunden gehalten.

Anschließend erfolgte die Bestimmung der Hydroxylzahl (OHZ) des entstandenen Oligocarbonates als Maß für die Aktivität des Katalysator gemäß DIN 53240-2. Unter Hydroxylzahl wird hierbei diejenige Menge Kaliumhydroxid in mg verstanden, die der bei der Acetylierung von 1 g Substanz gebundenen Menge Essigsäure äquivalent ist. Die Hydroxylzahl hat damit die Einheit mg KOH/g. (vgl. DIN-53240-2)

Der Wert der Hydroxylzahl ist somit umgekehrt proportional zur Aktivität des eingesetzten Katalysators.

**Tabelle 1:**

| **Katalysator (Yb(acac)**_{**3**}**)** | **Temp. (°C)** | **Zeit (h)** | **Druck (mbar)** | **Ausbeute (g)** | **OHZ** ^{**[1]**} **(mg KOH/g)** |
|---|---|---|---|---|---|
| Vergleichsbeispiel 1 | 50 | 5 | 20 | 203 | 227 |
| Beispiel 1 a) | 50 | 5 | 20 | 198 | 123 |
| | 100 | 36 | | | |
| Beispiel 1b) | 50 | 5 | 20 | 196 | 100 |
| | 100 | 60 | | | |

| | | | | | |
|---|---|---|---|---|---|
| [1] Die gemessene OH-Zahl (OHZ) ist umgekehrt proportional zur Aktivität des Katalysators und somit ein Maß für dessen Aktivität. Die OHZ und damit indirekt die Aktivität des Ytterbium(III)acetylacetonats wurde jeweils durch den vorangehend beschriebenen anwendungstechnischen Test ermittelt. | | | | | |

Die erfindungsgemäßen Beispiele 1 a) und b) zeigen, dass bei einer Trocknung bei wenigstens einer Temperatur > 50°C eine erhebliche Aktivitätssteigerung im Vergleich einem Katalysator, der lediglich bei 50°C getrocknet wurde, zu beobachten ist. Des Weiteren zeigen die Beispiele 1 a) und b), dass mit zunehmender Trocknungsdauer bei der erhöhten Temperatur noch eine weitere deutliche Aktivitätssteigerung erzielt werden kann.

### Vergleichsbeispiel 2:

Herstellung von Ytterbium(III)acetylacetonat mit einem Acetylaceton/YbCl₃-Verhältnis von 3,1/1,0:
a) In einem 2000 ml Doppelwand-Glasreaktor mit Thermometer und pH-Elektrode wurden 688 g Wasser, 200 g (0.516 mol, 1.0 Äquivalente) Ytterbium(III)chlorid-hexahydrat und 163 g (1.600 mol, 3.1 Äquivalente) Acetylaceton bei Raumtemperatur (20°C) vorgelegt. Innerhalb einer Stunde wurden 105 g (1.600 mol, 3.1 Äquivalente) einer 26 %igen wässrigen Ammoniaklösung zugetropft. Während der Zugabe stieg die Temperatur auf 33°C und der pH-Wert auf 8.49. Es wurde dann noch eine Stunde nachgerührt bevor auf 4°C abgekühlt wurde. Filtration und Waschen mit zweimal 250 ml Wasser lieferte 319 g Rohmaterial. Dieses Rohmaterial wurde anschließend bei 20 mbar 12 h bei 50°C und für 24 h bei 100°C getrocknet und die Ausbeute bestimmt (Tab.2).
b) Es wurde Ytterbium(III)acetylacetonat wie unter a) beschrieben hergestellt mit der Änderung, dass das Rohmaterial bei 20 mbar für 12 h bei 50°C und für 48 h bei 100°C getrocknet wurde.

Nach erfolgter Trocknung wurde jeweils die katalytische Wirksamkeit (Aktivität) des erhaltenen Ytterbium(III)acetylacetonats wie unter "Bestimmung der katalytischen Wirksamkeit (Aktivität)" beschrieben bestimmt. Die Ergebnisse sind in Tabelle 2 aufgeführt.

**Tabelle 2:**

| **Katalysator (Yb(acac)**_{**3**}**)** | **Temp. (°C)** | **Zeit (h)** | **Druck (mbar)** | **Ausbeute (g)** | **OHZ** ^{**[1]**} **(mg KOH/g)** |
|---|---|---|---|---|---|
| Vergleichsbeispiel 2 a) | 50 | 12 | 20 | 212 | 145 |
| | 100 | 24 | | | |
| Vergleichsbeispiel 2 b) | 50 | 12 | 20 | 208 | 153 |
| | 100 | 48 | | | |

| | | | | | |
|---|---|---|---|---|---|
| [1] vgl. Tab.1 | | | | | |

Die Vergleichsbeispiele 2 a) und b) zeigen, dass bei Einsatz von mehr als 3 Äquivalenten Acetylaceton bezogen auf 1 Äquivalent YbCl₃ auch mit Trocknungstemperaturen von > 50°C keine solche Aktivitässteigerung wie bei den erfindungsgemäßen Katalysatoren aus Beispiel 1 a) und b) beobachtet werden kann. Des Weiteren zeigen die Vergleichsbeispiele 2 a) und b), dass auch bei verlängerter Trocknungsdauer hier keine signifikante Aktivitätssteigerung erzielt werden konnte.

## Patentansprüche

1. Verfahren zur Herstellung eines Ytterbium(III)-β-diketonats durch Umsetzung wenigstens eines Ytterbium(III)-Salzes, wenigstens eines 1,3-Diketons und einer Base in wässriger Lösung, **dadurch gekennzeichnet, dass** weniger als 3 Äquivalente 1,3-Diketon bezogen auf 1 Äquivalent Ytterbium(III)-Salz eingesetzt werden und das erhaltene Ytterbium(III)-β-diketonat bei wenigstens einer Temperatur von >50°C bis 150°C länger als 5 Stunden getrocknet wird.

2. Verfahren gemäß Anspruch 1, **dadurch gekennzeichnet, dass** als Ytterbium(III)-Salz Ytterbium(III)-chlorid eingesetzt wird.

3. Verfahren gemäß Anspruch 1 oder 2, **dadurch gekennzeichnet, dass** als 1,3-Diketon Acetylaceton eingesetzt wird.

4. Verfahren gemäß wenigstens einem der Ansprüche 1 bis 3, **dadurch gekennzeichnet, dass** als Base Ammoniak eingesetzt wird.

5. Verfahren gemäß wenigstens einem der Ansprüche 1 bis 4, **dadurch gekennzeichnet, dass** die Trocknungszeit 10 bis 48 h beträgt.

6. Verfahren gemäß wenigstens einem der Ansprüche 1 bis 5, **dadurch gekennzeichnet, dass** 2,5 bis 2,99 Äquivalente 1,3-Diketon bezogen auf 1 Äquivalent Ytterbium(III)-Salz eingesetzt werden.

7. Verfahren gemäß wenigstens einem der Ansprüche 1 bis 6, **dadurch gekennzeichnet, dass** die Umsetzung bei wenigstens einer Temperatur von 15°C bis 60°C durchgeführt wird.

8. Verfahren gemäß wenigstens einem der Ansprüche 1 bis 7, **dadurch gekennzeichnet, dass** die Trocknung bei wenigstens einem Druck ≤ 1 bar durchgeführt wird.

9. Ytterbium(III)-β-diketonat hergestellt nach einem Verfahren gemäß wenigstens einem der Ansprüche 1 bis 8.

10. Verwendung von Ytterbium(III)-β-diketonat gemäß Anspruch 9 als Umesterungskatalysator.
